(19) Europäisches Patentamt
European Patent Office
Office européen des brevets

(11) Numéro de publication : **0 604 256 A1**

# (12) DEMANDE DE BREVET EUROPEEN

(21) Numéro de dépôt : **93402932.3**

(51) Int. Cl.$^5$ : **C07C 17/20,** C07C 19/08

(22) Date de dépôt : **03.12.93**

(30) Priorité : **17.12.92 FR 9215228**

(43) Date de publication de la demande : **29.06.94 Bulletin 94/26**

(84) Etats contractants désignés : **BE DE FR GB IT NL**

(71) Demandeur : **ELF ATOCHEM S.A.**
**4 & 8, Cours Michelet**
**La Défense 10**
**F-92800 Puteaux (FR)**

(72) Inventeur : **Oliveros Braun, Esther**
**Eichelstrasse 16**
**D-6749 Barbelroth (DE)**
Inventeur : **Wohlers, Michel**
**Buchenring 15B**
**D-7513 Buchig/Stutensee (DE)**
Inventeur : **Drivon, Gilles**
**3 Avenue Joanny Courbières**
**F-69850 Saint Martin en Haut (FR)**
Inventeur : **Kervennal, Jacques**
**134 Rue E. Locard**
**F-69005 Lyon (FR)**

(74) Mandataire : **Leboulenger, Jean et al**
**Elf Atochem S.A.**
**Département Propriété Industrielle, Cedex 42 - La Défense 10**
**F-92091 Paris la Défense (FR)**

## (54) Synthèse d'un bromure de perfluoroalkyle par bromation photochimique de l'iodure correspondant.

(57) L'invention concerne la préparation d'un bromure de perfluoroalkyle $R_FBr$ par bromation photochimique de l'iodure correspondant $R_FI$ en solution dans un solvant inerte.

La bromation est initiée par la coupure homolytique de la liaison C-I du composé $R_FI$, en présence de brome. Pour cela, on utilise une lampe à rayonnement particulier telle qu'une lampe à excimère $XeCl^*$ ou $I_2^*$.

EP 0 604 256 A1

La présente invention concerne le domaine des bromures $R_FBr$, $R_F$ désignant un radical perfluoroalkyle, linéaire ou ramifié, contenant de 2 à 14 atomes de carbone. Elle a plus particulièrement pour objet la préparation du bromure de n.perfluorooctyle $C_8F_{17}Br$ (ci-après désigné par l'abréviation PFOB).

On sait que les composés $R_FBr$ et plus particulièrement le PFOB présentent des propriétés très intéressantes dans le domaine médical comme agents de contraste (radiologie) ou comme transporteurs d'oxygène (sang artificiel). Leur futur développement dans ces applications apparaît être important. Aussi est-il nécessaire d'être en mesure de les produire industriellement par un procédé économique avec une très grande pureté.

Parmi les méthodes connues pour préparer les composés $R_FBr$, on peut d'abord signaler :

- l'action du brome sur un composé $R_FSF_5$ à 500°C en présence de nickel (brevet US 3 456 024) ;
- la photolyse en phase gazeuse d'un composé $R_FH$ par Br-Cl ou Br-F (J.L. Adcock et al, CA 100, 34092 e) ou par $Br_2$ (brevet FR 1 512 068).

La faiblesse des rendements obtenus et/ou l'utilisation de dérivés fluorés non disponibles industriellement ne permettent pas une production économique des composés $R_FBr$ à l'échelle industrielle.

Dans ses brevets EP 0 298 870 et 0 429 331 la Demanderesse a décrit des procédés de fabrication des composés $R_FBr$ à partir des chlorures de perfluoroalcane-sulfonyle $R_FSO_2Cl$ correspondants que l'on fait réagir soit avec HBr gazeux en présence d'un catalyseur (EP 0 298 870), soit avec un bromure d'ammonium ou de phosphonium quaternaire (EP 0 429 331). Les rendements obtenus sont généralement élevés, mais le sulfochlorure $R_FSO_2Cl$ utilisé est une matière première déjà très élaborée puisque sa synthèse à partir de l'iodure correspondant $R_FI$ nécessite deux étapes réactionnelles suivant l'équation :

$$2\ R_FI + 2\ SO_2 \xrightarrow{Zn} (R_FSO_2)_2Zn \xrightarrow{2Cl_2} 2\ R_FSO_2Cl + ZnCl_2$$

La voie la plus directe d'obtention des composés $R_FBr$ consiste évidemment en une bromation radicalaire des iodures correspondants $R_FI$, ces derniers étant des produits disponibles en quantités industrielles.

Dans International Journal of Chemical Kinetics, Vol. II, 273-285 (1975), E.N. Okafo et E. Whittle décrivent la cinétique de bromation thermique de $CF_3I$ dans un réacteur photochimique entre 173 et 321°C, en vue de déterminer l'énergie de dissociation de la liaison C-I.

Dans J. Chem. Soc. 1953, 3761-8, R.N. Haszeldine a décrit un procédé photochimique de réaction des $R_FI$ avec du brome en opérant en tube scellé avec un excès de brome (10 %) et en irradiant par une lumière UV pendant sept jours. La température réactionnelle et la pureté obtenue ne sont pas précisées ; il est simplement indiqué que le rendement est supérieur ou égal à 90 % selon la longueur de la chaîne perfluorée $R_F$.

Dans les exemples de la demande japonaise Kokai 85-184033 qui décrit la réaction des $R_FI$ avec $Br_2$ en présence d'un initiateur chimique de radicaux, les rendements indiqués ne dépassent pas 42 %.

La faiblesse des rendements obtenus et/ou la cinétique lente de ces techniques, ainsi que la transformation toujours incomplète du $R_FI$, ne permettent pas d'envisager une exploitation industrielle. En effet, compte tenu des applications visées (domaine médical), les composés $R_FBr$ et plus spécifiquement le PFOB doivent présenter une grande pureté, en particulier une très faible teneur en iodures $R_FI$ qui sont instables dans le temps avec développement d'une coloration rosée et difficilement séparables par distillation, leur point d'ébullition étant proche de celui du bromure correspondant.

D'autre part, comme indiqué dans la demande de brevet FR 2 677 979, il s'avère que, si l'on procède à la photobromation d'un $R_FI$ selon la technique classique, c'est-à-dire en introduisant le $R_FI$ et un excès de $Br_2$ dans un réacteur photochimique muni d'une lampe émettant dans les longueurs d'onde comprises entre 300 et 650 nm (cas des lampes à vapeur de mercure), le $R_FI$ se transforme en $R_FBr$ avec une cinétique assez rapide au début, puis la vitesse de la réaction diminue très rapidement pour s'arrêter à un taux de transformation global inférieur à 20 %. Pour obtenir une conversion pratiquement complète des $R_FI$, la demande de brevet précitée préconise, pour ce type de lampe, d'effectuer la photobromation en milieu dilué dans un solvant ($R_FBr$, $CCl_4$ ou $C_2F_3Cl_3$), la concentration en $R_FI$ de la solution diluée étant au plus égale à 0,5 mole/litre, de préférence inférieure à 0,35 mole/litre et avantageusement inférieure à 0,2 mole/litre. Bien que cette technique conduise à une conversion quasi-quantitative du $R_FI$, la cinétique de la réaction est globalement lente.

Dans la photobromation classique utilisant des rayonnements UV fournis par une lampe du type vapeur de mercure, le terme "photobromation" s'applique toujours à la coupure de la molécule de brome en radicaux Br· suivant la réaction :

$$Br_2 \xrightarrow{UV} 2\ Br\cdot \qquad (1)$$

ces radicaux réagissant ensuite sur le $R_FI$ de la manière suivante :

$$R_FI + Br\cdot \longrightarrow R_F\cdot + IBr$$
$$R_F\cdot + Br_2 \longrightarrow R_FBr + Br\cdot$$
$$I\cdot + Br\cdot \longrightarrow IBr \rightleftharpoons ½\, I_2 + ½\, Br_2$$

mais, du fait du large domaine spectral de ce type de lampe, une grande partie de la puissance de la lampe se dissipe pour conduire aux réactions suivantes :

$$IBr \rightarrow I\cdot + Br\cdot \qquad (2)$$
$$I_2 \rightarrow 2I\cdot \qquad (3)$$
$$R_FI \rightarrow R_F\cdot + I \qquad (4)$$

Si la réaction (4) n'est pas prépondérante, surtout en fin de réaction puisque la concentration résiduelle en $R_FI$ diminue au fur et à mesure de l'avancement de la réaction, il n'en est pas de même pour les réactions (2) et (3) qui deviennent prépondérantes par rapport à (1) du fait des concentrations relatives de $Br_2$, $I_2$ et IBr.

Il a maintenant été trouvé qu'on peut considérablement augmenter la vitesse de conversion du $R_FI$ en $R_FBr$ si l'on utilise une lampe irradiant exclusivement dans le domaine d'absorption du produit à bromer de manière à réaliser sélectivement la réaction (4) qui se poursuivra de la manière suivante :

$$R_FI \longrightarrow R_F\cdot + I\cdot \qquad (4)$$
$$R_F\cdot + Br_2 \longrightarrow R_FBr + Br\cdot$$
$$Br\cdot + R_FI \longrightarrow R_F\cdot + IBr$$
$$Br\cdot + I\cdot \longrightarrow I\ Br$$

Bien que la réaction globale soit identique dans les deux cas :

$$R_FI + Br_2 \rightarrow R_FBr + I\ Br$$

le procédé selon l'invention permet de façon inattendue d'obtenir, à puissance sensiblement égale de lampe, une cinétique réactionnelle au moins 5 fois supérieure qui se traduit industriellement par un important gain de productivité.

Pour obtenir conformément à la présente invention la coupure sélective du composé $R_FI$, on peut utiliser toute technique permettant ce type de réaction, en particulier toute lampe irradiant exclusivement entre 280 et 350 nm. A titre d'exemples non limitatifs de lampes utilisables, on peut mentionner plus particulièrement les lampes à excimère XeCl* ou $I_2$* dont le rayonnement se situe respectivement à 308 nm et 342 nm (B.Gellert et al, Appl. Phys. B 52, 14-21, 1991 et U.Kogelschatz, 9th Int. Symp. on Plasma Chemistry, Pugnochiuso, Italie 1989).

Comme dans la demande de brevet FR 2 677 979, la photobromation selon l'invention est effectuée en milieu dilué en utilisant comme solvant pour le $R_FI$, soit le composé $R_FBr$ lui-même, soit un solvant inerte tel que, par exemple, $CCl_4$, $C_2F_3Cl_3$ ou des composés $R_FH$ qui sont inertes à la bromation dans les conditions utilisées. Bien que le procédé selon l'invention permette d'opérer à une concentration en $R_FI$ qui peut aller jusqu'à 1 mole/litre et est donc supérieure à la limite de 0,5 mole/litre indiquée dans la demande FR 2 677 979, on travaillera avantageusement à une concentration en $R_FI$ inférieure à 0,70 mole/litre.

La solution de départ peut être préparée par simple mélange du $R_FI$ et du solvant, mais on peut également utiliser le produit issu de la bromation thermique ménagée de l'iodure $R_FI$, ce produit pouvant éventuellement être dilué par addition de $R_FBr$ pour l'amener à la concentration désirée en $R_FI$.

La photobromation peut être effectuée en discontinu ou en continu, dans tout réacteur photochimique, par exemple dans un photoréacteur à immersion ou à film tombant, équipé d'une lampe selon l'invention avec un manchon protecteur adapté, par exemple en borosilicate (Pyrex) pour une lampe à excimère $I_2$* et en quartz ou en quartz dopé par du titane (Germisil) pour une lampe à excimère XeCl*.

Le brome est de préférence introduit progressivement, de manière continue ou discontinue . La quantité totale de brome à utiliser est d'au moins une mole par mole de $R_FI$ présent dans la solution initiale, mais on préfère utiliser un excès de brome de 10 à 100 %.

La photobromation selon l'invention peut être menée à une température comprise entre 10°C et le point d'ébullition du solvant, mais on opère de préférence entre 10 et 80°C. L'opération est avantageusement réalisée à pression atmosphérique, mais on ne sortirait pas du cadre de la présente invention en travaillant sous légère pression ou dépression.

Le procédé selon l'invention s'applique aussi bien à la préparation d'un $R_FBr$ spécifique (par exemple $C_6F_{13}Br$, $C_8F_{17}Br$, $C_{10}F_{21}Br$, ...) qu'à celle d'un mélange de différents $R_FBr$ à partir d'un mélange des $R_FI$ correspondants.

Les exemples suivants illustrent l'invention sans la limiter. Pour suivre l'évolution de la réaction au cours du temps, des échantillons sont prélevés et analysés par chromatographie en phase gazeuse après traitement préalable par une solution aqueuse de sulfite de sodium.

## EXEMPLE 1

Cet exemple de photobromation des iodures de n.perfluorooctyle $C_8F_{17}I$ et de n.perfluorodécyle $C_{10}F_{21}I$ en solution dans le PFOB a été effectué en mode discontinu dans un réacteur photochimique à immersion, d'une capacité utile d'environ 1 litre, représenté sur la figure unique annexée. Un manchon (5) protège la lampe (3) avec ses électrodes externe (4) et interne (7) dans laquelle circule de l'eau désionisée à environ 15°C. Le réacteur comprend en outre une arrivée de brome, ainsi qu'un barreau magnétique d'agitation (1), un capillaire avec embout fritté (2) pour l'introduction d'azote permettant d'éliminer l'oxygène dissous et d'améliorer l'agitation, et un condenseur (6) à circulation d'eau industrielle (environ 13°C) pour éviter les pertes de solvant par évaporation.

On utilise une lampe à excimère XeCl* irradiant à 308 nm (puissance : 150 watts ; fabricant Asea Brown Boveri) munie d'un manchon en Germisil.

On charge le réacteur avec 1 litre d'une solution de PFOB contenant en poids environ 0,5 % de $C_8F_{17}I$, 0,5 % de $C_{10}F_{21}I$ et 3,5 % de $C_{10}F_{21}Br$, puis on procède à l'irradiation tout en introduisant du brome par petites quantités.

Durant l'opération, la température du mélange réactionnel est maintenue à environ 45°C.

Les résultats obtenus sont reportés dans le Tableau I ci-après.

## EXEMPLE 2 (Comparatif)

On opère avec un appareillage utilisant la même géométrie d'irradiation que pour l'exemple 1 et à partir de la même solution de iodures, mais on utilise une lampe à vapeur de mercure moyenne pression (Philips HPK 125) dont le domaine d'irradiation va de 300 à 650 nm et dont la raie la plus intense se situe à 367 nm. Cette lampe est munie d'un manchon en Pyrex.

Durant l'opération, la température du mélange réactionnel est maintenue à environ 45°C.

Les résultats obtenus sont reportés dans le tableau I suivant. Leur examen permet d'apprécier la différence de cinétique réactionnelle selon que l'on utilise une lampe à excimère XeCl* (Exemple 1 selon l'invention) ou une lampe UV classique (Exemple 2 comparatif). Avec la lampe à excimère XeCl*, on obtient une teneur en $R_FI$ résiduelle inférieure à 100 ppm en un temps environ 5 à 6 fois plus court qu'avec la lampe Hg.

**TABLEAU I**

| **Durée d'irradiation** (minutes) | **[$C_8F_{17}I$]ppm** | | **[$C_{10}F_{21}I$]ppm** | |
|---|---|---|---|---|
| | Exemple 1 Lampe XeCl* | Exemple 2 Lampe Hg | Exemple 1 Lampe XeCl* | Exemple 2 Lampe Hg |
| 0 (départ) | 4750 | 4750 | 5010 | 5010 |
| 30 | 3600 | - | 3200 | - |
| 60 | 1950 | 4300 | 2100 | 4500 |
| 120 | 820 | - | 850 | - |
| 180 | 350 | 3500 | 340 | 3800 |
| 240 | 150 | - | 140 | - |
| 360 | 60 | 2300 | 50 | 2500 |
| 580 | | 2000 | | 2100 |
| 1300 | | 400 | | 400 |
| 1920 | | 120 | | 110 |
| 2260 | | 60 | | 50 |

**EXEMPLE 3**

Dans le même réacteur qu'à l'exemple 1, équipé d'une lampe à excimère XeCl∗, on charge 1057 ml d'une solution de PFOB contenant 365 g (0,67 mole) de $C_8F_{17}I$ (soit une concentration molaire de 0,63 mole/litre) et 45 ml de $C_8F_{17}H$ comme étalon interne, puis on irradie à 45°C, tout en introduisant par petites quantités 61 ml de brome ce qui correspond à un excès d'environ 70 %.

Le tableau II suivant montre l'évolution du taux de conversion en fonction du temps d'irradiation. Malgré la concentration initiale élevée en iodure, l'emploi de la lampe XeCl∗ permet d'aboutir à une conversion quasi totale du $R_FI$.

**TABLEAU II**

| **Durée d'irradiation (heures)** | **[$C_8F_{17}I$] résiduel** | **Taux de conversion (%)** |
|---|---|---|
| 0 | 17,9 % | 0 |
| 20 | 8,45 % | 52,8 |
| 32 | 5,23 % | 70,8 |
| 53 | 0,88 % | 95,1 |
| 68 | 1250 ppm | 99,3 |
| 73 | < 100 ppm | 100 |

## Revendications

1. Procédé de préparation d'un bromure $R_FBr$ où $R_F$ désigne un radical perfluoroalkyle linéaire ou ramifié, contenant 2 à 14 atomes de carbone, par bromation photochimique de l'iodure de perfluoroalkyle $R_FI$ correspondant en solution dans un solvant inerte, caractérisé en ce que l'on utilise une lampe dont le rayonnement coupe sélectivement le composé $R_FI$ en radicaux $R_F\cdot$ et $I\cdot$

2. Procédé selon la revendication 1, dans lequel on utilise une lampe irradiant exclusivement entre 280 et 350 nm.

3. Procédé selon la revendication 2, dans lequel on utilise une lampe à excimère XeCl* ou à excimère $I_2$*.

4. Procédé selon l'une des revendications 1 à 3, dans lequel la concentration initiale en $R_FI$ de la solution est inférieure à 0,70 mole/litre.

5. Procédé selon l'une des revendications 1 à 4, dans lequel le solvant utilisé est le $R_FBr$ lui-même.

6. Procédé selon la revendication 5, dans lequel la solution initiale de $R_FI$ dans le $R_FBr$ est obtenue préalablement par bromation thermique du $R_FI$.

7. Procédé selon l'une des revendications 1 à 6, dans lequel on utilise au total 1 à 2 moles de brome par mole de $R_FI$ présent dans la solution initiale.

8. Procédé selon l'une des revendications 1 à 7, dans lequel la bromation photochimique est effectuée à pression atmosphérique et à une température comprise entre 10 et 80°C.

9. Application du procédé selon l'une des revendications 1 à 8 à la synthèse du bromure de n.perfluorooctyle.

10. Application du procédé selon l'une des revendications 1 à 8 à la synthèse du bromure de n.perfluorodécyle.

11. Application du procédé selon l'une des revendications 1 à 8 à la synthèse d'un mélange de bromures de perfluoroalkyle.

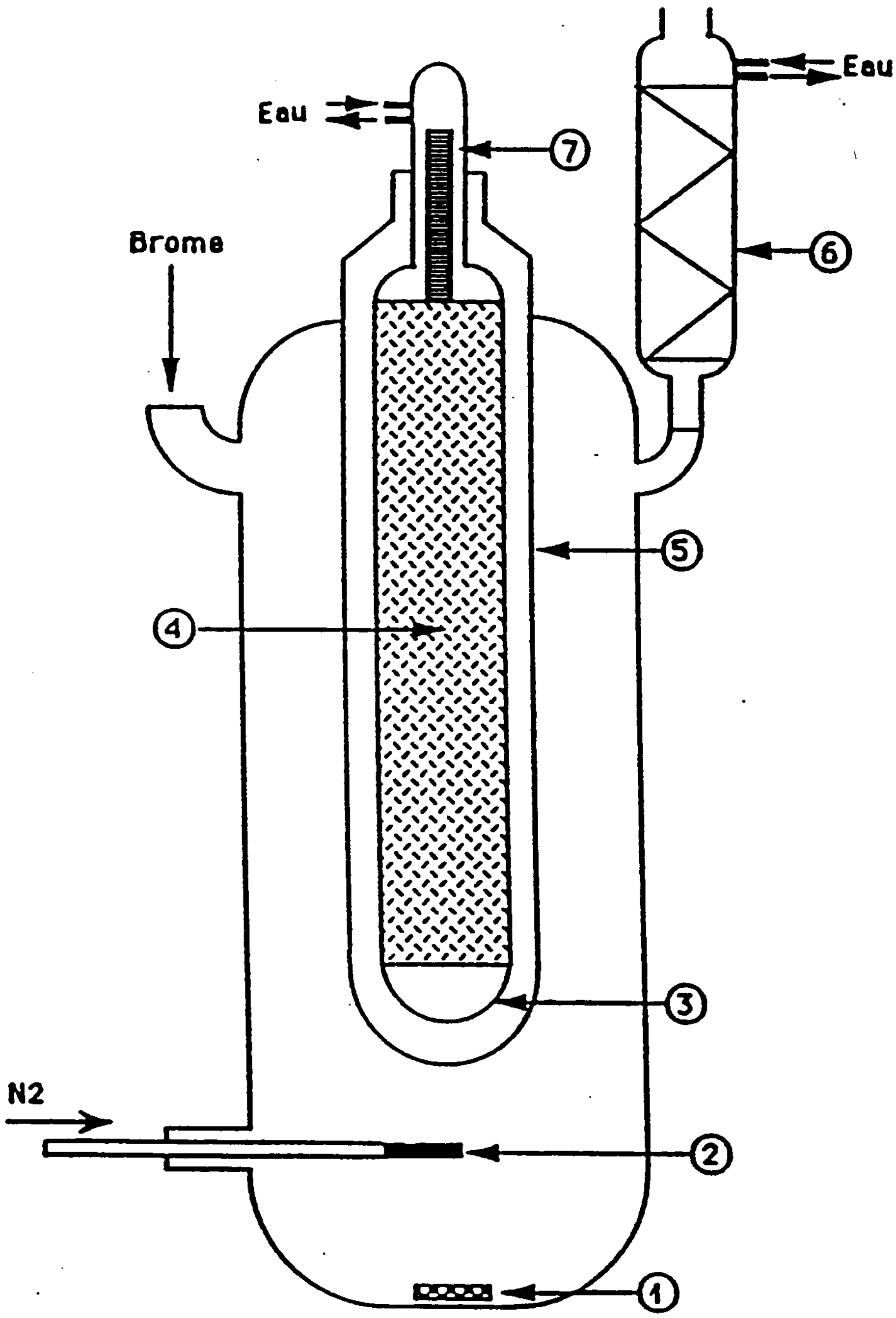
Eau
Eau
Brome
7
6
5
4
3
N2
2
1

Office européen des brevets

# RAPPORT DE RECHERCHE EUROPEENNE

Numero de la demande
EP 93 40 2932

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | DE-A-40 37 169 (HOECHST)<br>* le document en entier * | 1-11 | C07C17/20<br>C07C19/08 |
| A | EP-A-0 513 783 (HOECHST)<br>* revendications; exemples 1,7 * | 1-11 | |
| D,A | JOURNAL OF THE CHEMICAL SOCIETY 1953 , LETCHWORTH GB<br>pages 3761 - 3768<br>R. N. HAZELDINE 'Reactions of Fluorocarbon Radicals. Part XII. The Synthesis of Fluorocarbons and of Fully Fluorinated Iodo-, Bromo-, and Chloroalkanes.'<br>* page 3766, ligne 58 - page 3767, ligne 10 * | 1-11 | |
| | | | DOMAINES TECHNIQUES RECHERCHES (Int.Cl.5) |
| | | | C07C |

Le présent rapport a été établi pour toutes les revendications

| Lieu de la recherche | Date d'achèvement de la recherche | Examinateur |
|---|---|---|
| LA HAYE | 25 Février 1994 | Zervas, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)